# EUROPEAN PATENT APPLICATION

(11) **EP 3 693 021 A1**
(43) Date of publication of application: **12.08.2020**
(21) Application number: 20167724.2
(22) Date of filing: 13.03.2014
(51) Int. Cl.: A61K 47/18, A61K 38/00, A61K 47/26, A61K 47/40, A61K 9/50

(54) **COMPOSITION OF A SUSTAINED-RELEASE DELIVERY AND METHOD OF STABILIZING PROTEINS DURING FABRICATION PROCESS**

(30) Priority: 14.03.2013 US 201361786035 P
(62) Divisional of application: 14717337.1
(71) Applicant: Allergan, Inc., Irvine, CA 92612 (US)
(72) Inventor: TRAN, Simon H., Garden Grove, CA California 92843 (US); WU, Cindy W., Foothill Ranch, CA California 92610 (US)
(74) Representative: Hoffmann Eitle

(57) **Abstract**

As described herein, controlled and sustained administration of a therapeutic agent, such as a protein through the administration of one or more microparticles, may improve treatment of conditions, such as undesirable ocular conditions. The microspheres can be formulated to provide a sustained release of a protein, while reducing the number of protein aggregates, achieving desirable release profiles, and increasing stability of the protein within the microsphere formulations.

## Description

### CROSS-REFERENCE TO RELATED APPLICATIONS

This application claims the benefit under 35 U.S.C. § 119(e) of U.S. Provisional Application No. 61/786,035 filed on March 14, 2013, the entire content of which is incorporated herein by reference.

### BACKGROUND

### Field

This disclosure relates generally to formulations for sustained delivery of active agents, for example, proteins.

### Description of the Related Art

Delivery of drugs to the body of a human of animal, specifically to the eye of a human or animal, including the retina, vitreous, and uveal tract is typically achieved by high systemic dosing intraocular injections of compositions including an active agent, such as a protein.

Challenges may be presented from the stabilization of an active agent, such as a protein in a suitable carrier, such as microspheres. However, the process of encapsulating a protein into a microsphere formulation often destabilizes the protein through many different stresses such as concentration, shear, and interfacial stress. The effect of these process stresses can cause protein denaturation and aggregation, leading to lowered bioavailability and potentially, adverse immune response.

In addition to process stresses, proteins encapsulated in a polymer matrix are often subjected to unfavorable conditions from physiological environments as well as micro-environments from the within a polymer matrix. At the physiological environment level, proteins are subjected to elevated temperatures, such as 37 °C and above. At the micro-environmental level, proteins are also subjected to a concentration effect at the initial hydration stage, the pH shift, and the hydrophobic effect of polymers used in the matrix. These environmental conditions can be unfavorable to a protein's long-term stability and ultimately limit their potency and availability through such events as aggregation, chemical modification, and fragmentation.

### SUMMARY

Disclosed herein are formulations, methods of making formulations, and methods of using formulations including encapsulated proteins within a polymer network with high efficiency and quality to ensure sustained delivery for an extended period of time.

In one embodiment, a composition of matter includes particles, the particles including a protein, a biodegradable polymer, and one or more excipients. The particles can be configured to deliver the protein for a period of time of not less than two months. In some embodiments, the biodegradable polymer comprises poly(d,l-lactide-co-glycolide), poly(d,l-lactide), and/or polyethylene glycol copolymers with an inherent viscosity in the range of 0.1 dL/g to 1.0 dL/g. According to some embodiments, the protein has a molecular weight in the range of 10 kDa to 150 kDa. In an embodiment, the one or more excipients include cyclodextrin, trehalose, arginine, or a combination thereof. According to an embodiment, the cyclodextrin is sulfobutylether-beta-cyclodextrin.

In another embodiment, an aqueous formulation includes a protein, a buffer, and one or more excipients selected from the group consisting of cyclodextrin, trehalose, and arginine. The one or more excipients can be present in the aqueous formulation at a molar ratio in the range of 10/1 to 200/1, with respect to the protein molar concentration.

### BRIEF DESCRIPTION OF THE DRAWINGS

These and other features will now be described with reference to the drawings summarized below. These drawings and the associated description are provided to illustrate one or more embodiments of the invention and not to limit the scope of the invention.
FIG. 1 illustrates a bar graph comparing the soluble protein recovery of example embodiments.
FIG. 2 depicts a bar graph comparing the soluble protein recovery of other example embodiments.
FIG. 3 illustrates a bar graph comparing the soluble protein recovery of yet other example embodiments.
FIG. 4 shows a graph illustrating the release profiles of example embodiments of microspheres.
FIG. 5 depicts a graph illustrating the release profiles of example embodiments of microspheres.
FIG. 6 shows a graph illustrating the release profiles of example embodiments of microspheres.
FIG. 7 depicts a graph illustrating the release profiles of example embodiments of microspheres.
FIG. 8 shows a graph illustrating the binding activity of example embodiments of microspheres.
FIG. 9 depicts a graph illustrating the release profiles of example embodiments of microspheres.

### DETAILED DESCRIPTION

As described herein, controlled and sustained administration of a therapeutic agent, such as a protein through the administration of one or more microparticles, may improve treatment of conditions, such as undesirable ocular conditions of the anterior or posterior segment. The microparticles comprise a pharmaceutically acceptable polymeric composition and are formulated to release one or more pharmaceutically active agents, such as a protein, or other intraocular pressure lowering or neuroprotective agent, over an extended period of time. The microspheres can be formulated to provide a sustained release of a protein, while reducing the number of protein aggregates, achieving desirable release profiles, and increasing stability of the protein within the microsphere formulations.

The following terms as defined as follows, unless the context of the word indicates a different meaning.

As used herein, a "microsphere" or "microparticle" are interchangeably used to refer to particles, microspheres, microparticles, fine powders and the like comprising a biocompatible matrix encapsulating or incorporating a therapeutic component. According to some embodiments, microspheres do not include nanoparticles or nanospheres. Microspheres are generally biocompatible with physiological conditions of an organ of a human, such as an eye, and do not cause adverse side effects. Microspheres administered subconjunctivally may be used safely without disrupting vision of the eye. In some embodiments, microspheres have a maximum dimension, such as diameter or length, less than 1 mm. For example, microparticles can have a maximum dimension less than about 500 µm. Microparticles may also have a maximum dimension no greater than about 200 µm, or may have a maximum dimension from about 30 µm to about 50 µm, among other sizes.

As used herein, a "therapeutic component" refers to that portion of an microsphere other than the polymer matrix comprising one or more therapeutic agents or substances used to treat a medical condition of the eye. The therapeutic component may be a discrete region of a microsphere, or it may be homogenously distributed throughout the microsphere. The therapeutic agents of the therapeutic component can comprise at least one protein, are typically ophthalmically acceptable, and are provided in a form that does not cause significant adverse reactions when the microsphere is placed in an eye.

As used herein, a "protein" shall have its common meaning as known in the art, and can refer to large biological molecules consisting of one or more chains of amino acids. Proteins can perform a vast array of functions within living organisms, including catalyzing metabolic reactions, replicating DNA, responding to stimuli, and transporting molecules from one location to another. Proteins may be linear, branched, or circular and may me chemically synthesized or naturally or recombinantly produced. In some embodiments, proteins may also include proteins that are modified, such as PEGylated proteins or post-translational modification of proteins.

As used herein, a "drug release sustaining component" refers to a portion of the microsphere that is effective to provide a sustained release of the therapeutic agents from the microsphere. A drug release sustaining component may be a biodegradable polymer matrix, or it may be a coating covering a core region of the microsphere that comprises a therapeutic component.

As used herein, "associated with" means mixed with, dispersed within, coupled to, covering, or surrounding.

The term "biodegradable polymer" refers to a polymer or polymers which degrade in vivo, and wherein erosion of the polymer or polymers over time occurs concurrent with or subsequent to release of the therapeutic agent. The terms "biodegradable" and "bioerodible" are equivalent and are used interchangeably herein. A biodegradable polymer may be a homopolymer, a copolymer, or a polymer comprising more than two different polymeric units.

The term "therapeutically effective amount" as used herein, refers to the level or amount of agent needed to treat a condition, such as an ocular condition, or reduce or prevent ocular injury or damage without causing significant negative or adverse side effects to the eye or a region of the eye. In view of the above, a therapeutically effective amount of a therapeutic agent, such as a protein, is an amount that is effective in reducing at least one symptom of an ocular condition.

Those skilled in the art will appreciate the meaning of various terms of degree used herein. For example, as used herein in the context of referring to an amount (e.g., "about 6%"), the term "about" represents an amount close to and including the stated amount that still performs a desired function or achieves a desired result, e.g. "about 6%" can include 6% and amounts close to 6% that still perform a desired function or achieve a desired result. For example, the term "about" can refer to an amount that is within less than 10% of, within less than 5% of, within less that 0.1% of, or within less than 0.01% of the stated amount.

### Proteins

According to some embodiments, a formulation or microsphere formulation contains a protein, also referred to herein as a protein component. The protein can be linear, branched, or circular. The protein can be a fusion protein. In some embodiments, the protein may be truncated relative to its naturally occurring form. The protein may comprise no more than a single amino acid chain or two or more amino acid chains. If the protein contains more than two amino acid chains, the two or more chains may be covalently or non-covalently associated with one another. For example, the amino acid chains may be associated through disulfide bonds, or the two or chains may be associated with one another by non-covalent forces only. According to some embodiments, the protein does not contain synthetically or post-translationally modified amino acids. The proteins may be produced recombinantly (e.g. by a mammalian or a prokaryotic cell culture), synthetically (e.g. by solid phase synthesis), or isolated from natural sources (e.g. mammalian or bacterial cell culture, plasma, serum, plant, fungus, or the like).

Non-limiting examples of proteins that can be included in formulations and microsphere formulations, and therefore delivered include monoclonal and polyclonal antibodies, bispecific antibodies, bispecific antibodies, antibody fragments, anticalins, DARPins, and enzymes. Other examples include glycoproteins and serum albumins. If used, a monoclonal or polyclonal antibody can be used in its natural form as produced, for example, by a cell and may or may not contain post-translational modifications, or may be used in a chemically or enzymatically modified form produced subsequent to its isolation from a cell culture or other biological sample. If used, an antibody may be chimeric. In some embodiments, the antibody may be an IgA, IgD, IgE, IgG, or IgM. Antibody fragments include those generated by papain (e.g., the Fab fragment) or pepsin cleavage of an antibody. More generally, useful antibody fragments include Fab', F(ab)2, Fabc, and Fv fragments. In some embodiments, a complement antibody fragment can be used. The antibody fragments may either be produced by the modification of whole antibodies or those synthesized de novo using recombinant DNA methodologies, and further include "humanized" antibodies made by now conventional techniques. "Antibody fragments" comprise a portion of a full length antibody, generally the antigen binding or variable domain thereof.

Some embodiments provide for a formulations and microsphere formulations comprising an anti-VEGF antibody or protein (i.e., an antibody or protein that specifically binds to vascular endothelial growth factor protein, or VEGF-A). Useful anti-VEGF antibodies include, but are not limited to, ranibizumab (LUCENTIS®) and bevacizumab (AVASTIN®). Useful anti-VEGF proteins include Aflibercept (Eylea®) (also known as VEGF TRAP), VEGF-binding DARPins, and VEGF-binding anticalins. VEGF Trap (Regeneron Pharmaceuticals, New York) is a fusion protein that contains portions of the extracellular domains of two different VEGF receptors connected to the Fc region (C-terminus) of a human antibody. In some embodiments the formulation or microsphere formulation may comprise an antibody selected from the group consisting of anti-VEGF antibodies, anti-VEGF receptor antibodies, anti-PDGF (platelet derived growth factor) antibodies, anti-integrin antibodies, therapeutically effective fragments thereof, and combinations thereof.

In another embodiment, formulations or microsphere formulations may include a protein comprising an anti-TNF (tumor necrosis factor) antibody or protein. Examples of anti-TNF antibodies include, but are not limited to, Adalimumab (HUMIRA®), Infliximab (REMICADE®), certolizumab pegol (CIMZIA®), and golimumab (SIMPONI®). Useful anti-TNF proteins include the fusion protein Etanercept (ENBREL®). Formulations and microsphere formulations including anti-TNF proteins may be particularly useful for treating uveitis and Behcet's disease.

According to some embodiments, proteins can include growth factors such as nerve growth factors, acid fibroblast growth factors, and basic fibroblast growth factors; neutrophic factors such as ciliary neurotrophic factor, brain-derived neurotrophic factor, and glial cell line-derived neurotrophic factor; cytokines such as interferon-gamma and interleukin-10; anti-proliferative compounds such as rituximab; and fibrinolysing protein such as tissue plasminogen activator. Therapeutic uses for such proteins may be generally known in the art.

Thus, according to some embodiments, formulations or microsphere formulations may comprise an antibody selected from the group consisting of anti-VEGF antibodies (antibodies that specifically bind to a VEGF), anti-PDGF antibodies, anti-VEGF receptor antibodies, anti-integrin antibodies, therapeutically effective fragments thereof, and combinations thereof.

Vascular endothelial growth factor A (VEGF-A, also referred to as VEGF), is a secreted mitogen specific for vascular endothelial cells that can stimulate endothelial cell growth in vitro and angiogenesis in vivo. VEGF-A can occur in different isoforms. All isoforms of VEGF-A, except VEGF-A₁₂₁, bind heparin. In humans the most abundant isoform of VEGF-A is a 165-amino acid polypeptide, VEGF-A₁₆₅. *See*, for example, Houck et al., Mol. Endocrin. 5:1806 (1991) and Leung et al., Science 246:1306 (1989).

Accordingly, some embodiments provide for a microsphere formulation comprising a biodegradable polymer matrix and a protein comprising, consisting essentially of, or consisting of an antibody, DARPin, or anticalin that binds to VEGF-A₁₆₅. The same antibody, DARPin, or anticalin may recognize and bind all isoforms of VEGF-A since the antibody, DARPin, or anticalin may recognize an epitope present in all isoforms of VEGF-A. For example, the antibody, DARPin, or anticalin may recognize and specifically bind an epitope in the region of the VEGF-A receptor binding domains present in all isoforms of VEGF-A, including VEGF₁₂₁. Accordingly, a single anti-VEGF antibody (or DARPin or anticalin), in a formulation or microsphere formulation may recognize and specifically bind all isoforms of VEGF-A. Antibodies, including monoclonal antibodies and humanized anti-VEGF antibodies, that bind to VEGF-A or VEGF receptor have been described. See, for example, U.S. Patent 5,955,311 and U.S. Patent 6,884,879. Bevacizumab is one example of a monoclonal antibody that specifically binds to and inhibits human VEGF-A. Inhibitory antibodies directed against PDGF have also been described. See, for example, WO 2003/025019. Thus, the microparticles may comprise a protein which comprises, consists essentially of, or consists of anticalin.

Formulations or microsphere formulations comprising a VEGF (vascular endothelial growth factor) or platelet-derived growth factor (PDGF) inhibitor or both, such as an antibody or combination of antibodies that specifically bind(s) VEGF and/or PDGF in vivo, may have particular utility for treating macular degeneration (including wet age-related macular degeneration), retinopathy, diabetic retinopathy, proliferative diabetic retinopathy, sickle cell retinopathy, retinopathy of prematurity, ischemic retinopathy, ocular neovascularization (abnormal growth of new blood vessels in the eye), choroidal neovascularization, neovascularization due to retinal vein occlusion, corneal neovascularization, diabetic retinal ischemia, and macular edema in a patient in need thereof.

In one embodiment, a formulation or microsphere formulation includes a biodegradable polymer matrix and first and second antibodies, or a bispecific antibody, that specifically bind(s) VEGF and PDGF, respectively, wherein the formulation or microsphere formulation provides continuous release of the first and second antibodies or bispecific antibody in a biologically active form for at least 30, 60, or 90 days after placement of the formulation or microsphere formulation in an eye of a mammal. In one form, the antibodies or bispecific antibody are specific for vascular endothelial growth factor-A (VEGF-A) and platelet-derived growth factor-B (PDGF-B). These formulation or microsphere formulations may be particular useful for treating ocular tumors, ocular neovascularization, choroidal neovascularization, and macular degeneration. By "PDGF-B" is meant a B chain polypeptide of PDGF.

The present formulations and microsphere formulations are designed to maintain the biological activity of the antibody, such that when the antibody is released from the system it will specifically bind to its designated protein target. The binding of the antibody to the protein target may provide interference between the protein and its ligand or receptor, and thus the function mediated by a protein/receptor interaction can be inhibited or reduced by the antibody. Several methods for determining whether an antibody specifically binds to or is "immunoreactive with" a protein (polypeptide) target are known in the art. Immuno chemiluminescence metric assays (ICMA), enzyme-linked immunosorbent assays (ELISA) and radioimmunoassays (RIA) are some examples.

In some embodiments, formulations and microsphere formulations can include a biodegradable polymer matrix and a protein, such as one or more monoclonal antibodies, bispecific antibodies, DARPins, anticalins, antibody fragments, recombinant polypeptides derived from an antibody variable region, or mixtures thereof, that interact(s) with (e.g., bind to and lessen or inhibit the activity of) VEGF or platelet-derived growth factor (PDFG) or both VEGF and PDFG. For example, according to an embodiment, a formulation or microsphere formulation comprises a biodegradable polymer matrix and a bispecific antibody that specifically binds VEGF and PDFG, wherein the formulation or microsphere formulation provides for continuous release of the antibody in a biologically active form for at least 90 days after placement in an eye of a mammal. In other embodiments formulations and microsphere formulations may comprise a biodegradable polymer matrix and an antibody specific for the VEGF receptor.

Monoclonal antibodies useful in formulations and microsphere formulations can be obtained using routine methods known to persons of ordinary skill in the art. Briefly, animals such as mice are injected with a desired target protein or portion thereof (the antigen), such as VEGF or VEGFR. The target protein can be coupled to a carrier protein. The animals are boosted with one or more target protein injections, and are hyperimmunized by an intravenous (IV) booster 3 days before fusion. Spleen cells from the *mice* are isolated and are fused by standard methods to myeloma cells. Hybridomas can be selected in standard hypoxanthine/aminopterin/thymine (HAT) medium, according to standard methods. Hybridomas secreting antibodies which recognize the target protein are identified, cultured, and subcloned using standard immunological techniques, and the antibody purified, for example, by affinity chromatography. In certain embodiments of the present delivery systems, an anti-VEGF or anti-VEGFR monoclonal antibody is obtained from ImClone Systems, Inc. (NY, NY). For example, the present formulations may include an antibody available from ImClone Systems under the name IMC-18F1, or an antibody under the name of IMC-1121 Fab. Another anti-VEGF antibody fragment that may be used in the present drug formulations is ranibizumab, a Fab fragment that binds VEGF-A. Another anti-VEGF antibody useful in the present drug delivery systems is bevacizumab, a monoclonal antibody that binds VEGF-A.

The protein component may be in a liquid, derivatized, particulate, or powder form and it may be entrapped by the biodegradable polymer matrix. Usually, protein particles in spray dried particle or in powder form will have an effective average particle size of less than about 1 micron. In some embodiments, the particles may have an effective average particle size about an order of magnitude smaller than 1000 nanometers. For example, the particles may have an effective average particle size of less than about 500 nanometers. In some microsphere formulations, the particles may have an-effective average particle size of less than about 250 nanometers, and in still further embodiments, a size less than about 200 nanometers.

According to some embodiments, the protein component may have a suitable molecular weight. The molecular weight of the protein can be in the range of about 10 kilodaltons ("kDa") to about 150 kDa. According to some embodiments, the molecular weight of the protein component can be greater than about 5 kDa, greater than 10 kDa, greater than 20 kDa, greater than 50 kDa, or greater than 100 kDa. According to some embodiments, the protein component can have a molecular weight in the range of about 10 kDa to 30 kDa, 14 kDa to 16 kDa, 14 kDa to 21 kDa, or 20 kDa to 50 kDa.

In some embodiments, the protein(s) according to formulations or microsphere formulation may be at least 20, at least 30, at least 50, at least 100, at least 200, or at least 300 amino acids in length. In some embodiments, the protein component comprises three or more amino acids. In some embodiments, the protein may be from 30 to 50 amino acids in length or from 100 to 500 amino acids in length.

A formulation or microsphere formulation, according to some embodiments, can comprise protein in an amount of 1.0% to about 50% by weight of the formulation, based on the total weight of the formulation or microsphere formulation. According to some embodiments, the protein can be present in a formulation or microsphere formulation in an amount of about 5% to about 30% by weight, about 5% to about 40% by weight, about 10% to about 25% by weight, or about 5% about 10%, about 20%, or about 30% by weight protein, based on the total weight of the formulation or microsphere formulation. According to some embodiments, the formulation or microsphere formulation comprises about 5% by weight, about 6% by weight, about 7% by weight protein, about 8% by weight protein, about 10% by weight protein, about 15% by weight protein, about 20% by weight protein, about 25% by weight protein, about 30% by weight protein, or about 35% by weight protein, based on the total weight of the microsphere formulation.

### Excipients

According to some embodiments, formulations or microsphere formulations can include one or more general class of excipients. General excipient classes can include albumins, buffer agents, amino acids, carbohydrates, chelating agents, cyclodextrins, polyethylene glycol, surfactants, and the like. According to some embodiments, compositions, including microsphere compositions include no more than three classes of excipient. According to some embodiments, compositions include only two classes of excipients.

Buffer agents may include phosphate buffers, such as dibasic sodium phosphate heptahydrate, monobasic sodium phosphate monohydrate; and/or borate buffers, such as sodium borate, boric acid, sodium chloride (according to Eu. Pharmacopeia). According to some embodiments, a buffer agent is the only excipient used in a formulation or a microsphere formulation. In some embodiments, a buffer agent is one of two or one of three excipients present in a formulation or microsphere formulation.

Certain embodiments of the present compositions comprise a cyclodextrin component. For example, the cyclodextrin component may be associated with the therapeutic component to enhance the solubility of the therapeutic component in the composition, enhance or improve the stability of the therapeutic component in the composition, relative to compositions which comprise the same therapeutic component and substantially no cyclodextrin component.

In certain embodiments, the excipient of the present compositions comprises a cyclodextrin component, such as one or more types of different cyclodextrins or cyclodextrin derivatives. For example, the cyclodextrin component may comprise a cyclodextrin selected from the group consisting of alpha-cyclodextrin, beta-cyclodextrin, gamma-cyclodextrin, derivatives thereof, and mixtures thereof. The term "cyclodextrin derivative" has the broadest meaning generally understood in the art, and refers to a compound or a mixture of compounds wherein one or more of the free hydroxyl groups of alpha-, beta-, or gamma-cyclodextrin is replaced with any other group. A "water-soluble" cyclodextrin derivative is soluble at a concentration of at least 300 mg/mL in water. The cyclodextrin derivative used in the compositions disclosed herein may vary. Derivatives of alpha-cyclodextrin, beta-cyclodextrin, and gamma-cyclodextrin may be used. In certain compositions, a beta-cyclodextrin derivative such as calcium sulfobutylether-beta-cyclodextrin, sodium sulfobutylether-beta-cyclodextrin, and hydroxypropyl-beta-cyclodextrin, may be used. Alternatively, a gamma-cyclodextrin derivative such as calcium sulfobutylether-gamma-cyclodextrin, sodium sulfobutylether-gamma-cyclodextrin, and hydroxypropyl-gamma-cyclodextrin may be used. Some specific derivatives contemplated herein are the hydroxypropyl derivatives of cyclodextrins, such as hydroxypropyl-beta-cyclodextrin or hydroxypropyl-gamma-cyclodextrin.

Cyclodextrins (CDs), are commercially available in compounds such as hydroxypropyl gamma-CD ("HP-gamma-CD"), commercially available as CAVASOL®, sulfobutyl ether 4 beta-CD ("SBE-CD"), commercially available as CAPTISOL®, and hydroxypropyl beta-CD ("HP-beta-CD"), commercially available as KLEPTOSE ®, hydroxypropyl-alpha-cyclodextrin ("HP-alpha-cyclodextrin"), and the like.

If used, a cyclodextrin can comprise a certain weight percentage of the disclosed formulations or microsphere formulations. According to some embodiments, the cyclodextrin is present in a formulation in an amount in the range of 10% to 75% by weight, based on the total weight of the formulation. According to some embodiments, the cyclodextrin is present in a formulation in an amount in the range of 30% to 70% by weight, 1% to 10% by weight, 3% by weight to 7% by weight, or 5% by weight to 15% by weight, based on the total weight of the formulation. According to some embodiments, a cyclodextrin is the only excipient used in a formulation or a microsphere formulation. In some embodiments, a cyclodextrin is one of two or one of three excipients present in a formulation or microsphere formulation. According to some embodiments, SBE-CD and a buffer agent are the only excipients present in a formulation or microsphere formulation.

In some embodiments, the one or more excipients can include an amino acid, separate from the protein component. Example excipient amino acids that can be used in formulations and microsphere formulation can include arginine, proline, aspartic acids, glutamic acids, lysine, histidine, and/or glycine as well as other amino acids known to those of skill in the art. According to some embodiments, a first amino acid is present in a formulation in an amount in the range of 0.5% by weight to 2% by weight, 1% by weight to 10% by weight, 1% by weight to 3% by weight, 4% by weight to 7% by weight, 3% by weight to 7% by weight, or 5% by weight to 15% by weight, based on the total weight of the formulation. According to some embodiments, a second amino acid is present in a formulation in an amount in the range of 0.5% by weight to 2% by weight, 1% by weight to 10% by weight, 1% by weight to 3% by weight, 4% by weight to 7% by weight, 3% by weight to 7% by weight, or 5% by weight to 15% by weight, based on the total weight of the formulation. According to some embodiments, an amino acid and a buffer agent are the only excipients present in a formulation or microsphere formulation. In some embodiments, an amino acid is one of two or one of three excipients present in a formulation or microsphere formulation.

In some embodiments, the one or more excipients can include a carbohydrate. Example excipient carbohydrates that can be used in formulations and microsphere formulation can include trehalose, inulin, or sucralose, as well as other carbohydrates known to those of skill in the art. According to some embodiments, a carbohydrate is present in a formulation in an amount in the range of 0.5% by weight to 2% by weight, 1% by weight to 10% by weight, 1% by weight to 3% by weight, 4% by weight to 7% by weight, 3% by weight to 7% by weight, or 5% by weight to 15% by weight, based on the total weight of the formulation. According to some embodiments, a carbohydrate and a buffer agent are the only excipients present in a formulation or microsphere formulation. In some embodiments, a carbohydrate is one of two or one of three excipients present in a formulation or microsphere formulation.

In some embodiments, the one or more excipients can include a chelating agent. An example excipient chelating agent that can be used in formulations and microsphere formulation can include EDTA. According to some embodiments, a chelating agent and a buffer agent are the only excipients present in a formulation or microsphere formulation. In some embodiments, a chelating agent is one of two or one of three excipients present in a formulation or microsphere formulation.

In some embodiments, the one or more excipients can include a polyethylene glycol. An example excipient polyethylene glycol that can be used in formulations and microsphere formulation can include PEG400. According to some embodiments, a polyethylene glycol and a buffer agent are the only excipients present in a formulation or microsphere formulation. In some embodiments, a polyethylene glycol is one of two or one of three excipients present in a formulation or microsphere formulation.

In some embodiments, the one or more excipients can include a surfactant. An example excipient surfactant that can be used in formulations and microsphere formulation can include polysorbate 80. According to some embodiments, a surfactant and a buffer agent are the only excipients present in a formulation or microsphere formulation. In some embodiments, a surfactant is one of two or one of three excipients present in a formulation or microsphere formulation.

According to some embodiments, an excipient component present in a composition can be in a liquid form with a suitable molar concentration. In some embodiments, one or more excipient present in the composition has a molar concentration in the range of about 6mM to about 120 mM. In some embodiments, the excipient has a concentration in the range of about 5 mM to about 75 mM. According to other embodiments, the excipient has a concentration in the range of about 6 mM to about 60 mM. According to yet other embodiments, the excipient has a concentration of about 6 mM, about 20 mM, about 40 mM, about 60 mM, about 100 mM, about 120 mM, and the like. According to an embodiment, an excipient, such as a cyclodextrin, such as sulfobutylether-beta-cyclodextrin can be used having a 60 mM concentration.

The excipient or excipients may be present in formulations or microsphere formulations in an amount of 0.01% by weight to about 50% by weight, 0.01% to about 20% by weight, about 0.01% by weight to about 15% by weight of the formulation, or about 15% by weight to about 30% by weight of the formulation, based on the total weight of the formulation.

### Biodegradable Polymer Matrix

In one embodiment, a microsphere comprises a biodegradable polymer matrix. The biodegradable polymer matrix is one type of a drug release sustaining component. The biodegradable polymer matrix can be made of one polymer or a mixture of two or more polymers. The biodegradable polymer matrix can be effective in forming a biodegradable microsphere. The biodegradable microsphere comprises a protein component associated with the biodegradable polymer matrix.

Suitable polymeric materials or compositions for use in the microspheres include those materials which are compatible, that is biocompatible, with the eye so as to cause no substantial interference with the functioning or physiology of the eye. Such materials can be at least partially or substantially completely biodegradable or bioerodible.

Examples of useful polymeric materials include, without limitation, such materials derived from and/or including organic esters and organic ethers, which when degraded result in physiologically acceptable degradation products, including the monomers. Also, polymeric materials derived from and/or including, anhydrides, amides, orthoesters and the like, by themselves or in combination with other monomers, may also find use. The polymeric materials may be addition or condensation polymers, advantageously condensation polymers. The polymeric materials may be cross-linked or non-cross-linked, for example not more than lightly cross-linked, such as less than about 5%, or less than about 1% of the polymeric material being cross-linked. For the most part, besides carbon and hydrogen, the polymers will include at least one of oxygen and nitrogen, advantageously oxygen. The oxygen may be present as oxy, e.g. hydroxy or ether, carbonyl, e.g. non-oxo-carbonyl, such as carboxylic acid ester, and the like. The nitrogen may be present as amide, cyano and amino. The polymers set forth in Heller, Biodegradable Polymers in Controlled Drug Delivery, In: CRC Critical Reviews in Therapeutic Drug Carrier Systems, Vol. 1, CRC Press, Boca Raton, Fla. 1987, pp 39-90, which describes encapsulation for controlled drug delivery, may find use in the present microspheres.

Of additional interest are polymers of hydroxyaliphatic carboxylic acids, either homopolymers or copolymers, and polysaccharides. Polyesters of interest include polymers of D-lactic acid, L-lactic acid, racemic lactic acid, glycolic acid, polycaprolactone, and combinations thereof. Generally, by employing the L-lactate or D-lactate, a slowly eroding polymer or polymeric material is achieved, while erosion is substantially enhanced with the lactate racemate.

Among the useful polysaccharides are, without limitation, calcium alginate, and functionalized celluloses, particularly carboxymethylcellulose esters characterized by being water insoluble, a molecular weight of about 5 kD to 500 kD, for example.

Other polymers of interest include, without limitations polyvinyl alcohol, polyesters, polyethers and combinations thereof which are biocompatible and may be biodegradable and/or bioerodible.

Some characteristics of the polymers or polymeric materials for use can include biocompatibility, compatibility with the protein, ease of use of the polymer in making the microspheres, a half-life in the physiological environment of at least about two weeks, in some embodiments greater than about one month, and solubility (or lack of solubility) in water.

The biodegradable polymeric materials included to form the microspheres can be subject to enzymatic or hydrolytic instability. Water soluble polymers may be cross-linked with hydrolytic or biodegradable unstable cross-links to provide useful water insoluble polymers. The degree of stability can be varied widely, depending upon the choice of monomer, whether a homopolymer or copolymer is employed, employing mixtures of polymers, and whether the polymer includes terminal acid groups.

In some embodiments, equally important to controlling the biodegradation of the polymer and hence the extended release profile of the microsphere formulation is the relative average molecular weight of the polymeric composition employed in the microspheres. Different molecular weights of the same or different polymeric compositions may be included in the microspheres to modulate the release profile. In certain formulations and microsphere formulations, the relative average molecular weight of the polymer can range from about 9 kD to about 64 kD, about 10 kD to about 54 kD, or from about 12 kD to about 45 kD.

In some microspheres, copolymers of glycolic acid and lactic acid are used, where the rate of biodegradation is controlled by the ratio of glycolic acid to lactic acid. The most rapidly degraded copolymer has roughly equal amounts of glycolic acid and lactic acid. Homopolymers, or copolymers having ratios other than equal, are more resistant to degradation. The ratio of glycolic acid to lactic acid will also affect the brittleness of the microspheres. The mol percentage (% mol) of polylactic acid in the polylactic acid polyglycolic acid (PLGA) copolymer can be between 15 mol% and about 85 mol%. In some embodiments, the mol percentage of polylactic acid in the (PLGA) copolymer is between about 35 mol% and about 65 mol %. In some embodiments, a PLGA copolymer with 50 mol% polylactic acid and 50 mol% polyglycolic acid can be used in the polymer matrix. In some embodiments, a PLGA copolymer with 75 mol% polylactic acid and 25 mol% polyglycolic acid can be used in the polymer matrix.

The biodegradable polymer matrix of the subconjunctival microspheres may comprise a mixture of two or more biodegradable polymers. For example, the microspheres may comprise a mixture of a first biodegradable polymer and a different second biodegradable polymer. One or more of the biodegradable polymers may have terminal acid groups.

According to some embodiments, the biodegradable polymer used may have a inherent viscosity in the range of about 0.1 dL/g to about 1.0 dL/g. According to some embodiments, the biodegradable polymer used may have a inherent viscosity in the range of about 0.5 dL/g to about 1.0 dL/g.

Some example polymers that may be used alone or in combination to form the microspheres include those listed in TABLE A below, the specifications, data sheets, and testing data of the commercially available polymers are incorporated by reference, in their entirety:

**TABLE A**

| Trade Name of Commercially Available Polymer (From EVONIK) | Polymer | Intrinsic Viscosity (dL/g) | Molecular Weight (low, medium, high) |
|---|---|---|---|
| RG502S | 50:50 poly (D, L-lactide-co-glycolide) | 0.16-0.24 | low |
| RG502H | 50:50 poly (D, L-lactide-co-glycolide), acid end capped | 0.16-0.24 | low |
| RG504 | 50:50 poly (D, L-lactide-co-glycolide) | 0.45-0.60 | medium |
| RG505 | 50:50 poly (D, L-lactide-co-glycolide) | 0.61-0.74 | medium |
| RG752S | 75:25 poly (D, L-lactide-co-glycolide) | 0.16-0.24 | low |
| RG755S | 75:25 poly (D, L-lactide-co-glycolide) | 0.50-0.70 | medium |
| RG858S | 85:15 poly (D, L-lactide-co-glycolide) | 1.3 - 1.7 | medium |
| R202H | poly (D, L-lactide), acid end capped | 0.16-0.24 | low |
| R203S | poly (D, L-lactide) | 0.25-0.35 | medium |
| R208 | poly (D, L-lactide) | 1.8 - 2.2 | high |
| RGPd5055 | Diblock PEGylated copolymer of PLGA w/ 5% w/w polyethylene glycol | | |

The biodegradable polymer microspheres can comprise a mixture of two or more biodegradable polymers. For example, the microspheres may comprise a mixture of a first biodegradable polymer and a different second biodegradable polymer. One or more of the biodegradable polymers may have terminal acid groups. According to some embodiment, microspheres include no more than one type of biodegradable polymer.

According to some embodiments, the biodegradable polymer comprises 75% by weight to 94% by weight of the microsphere formulation, based on the total weight of the formulation. According to other embodiments, the biodegradable polymer comprises 75% by weight to 85% by weight of the microsphere formulation, based on the total weight of the microsphere formulation. According to other embodiments, the biodegradable polymer comprises 85% by weight to 95% by weight of the microsphere formulation, based on the total weight of the microsphere formulation.

### Microspheres

Protein-containing microspheres can be made using suitable processes. In an embodiment, protein containing microsphere can be made by a water in oil in water emulsion process as described in PCT Publication No. 2011/041642, whose methods for making microspheres via a water in oil in water emulsion process are herein incorporated by reference in their entirety.

The release of the protein component from microspheres may include an initial burst of release followed by a gradual increase in the amount of the protein component released, or the release may include an initial delay in release of the protein component followed by an increase in release.

It may be desirable to provide a relatively constant rate of release of the protein component from the microspheres. For example, it may be desirable for the protein component to be released in amounts from about 2 µg to about 10 µg per day for the life of the microspheres. According to some embodiments, it may be desirable for the protein component to be released in an amount of about 4 µg per day, 5 µg per day, 6 µg per day, 7 µg per day, and the like. However, the release rate may change to either increase or decrease depending on the formulation of the biodegradable polymer matrix. In addition, the release profile of the protein component may include one or more linear portions and/or one or more non-linear portions. In some embodiments, the release rate is greater than zero once the microspheres have begun to degrade or erode.

The microspheres may be monolithic, i.e. having the active agent or agents homogenously distributed through the polymeric matrix, or encapsulated, where a reservoir of active agent is encapsulated by the polymeric matrix. Due to ease of manufacture, microspheres can exhibit an advantage over encapsulated forms. However, the greater control afforded by the encapsulated microspheres may be of benefit in some circumstances, where the therapeutic level of the drug falls within a narrow window. In addition, the therapeutic component, including the protein component, may be distributed in a non-homogenous pattern in the matrix. For example, the microspheres may include a portion that has a greater concentration of the protein component relative to a second portion of the microspheres.

According to some embodiments, the microspheres disclosed herein may have a size of between about 5 µm and about 1 mm, or between about 10 µm and about 0.2 mm for administration with a needle. According to an embodiment, the microspheres may have a size of about .15 mm. For needle-injected microspheres, the microsphere may have any appropriate dimensions so long as the longest dimension of the microsphere permits the microsphere to move through an administration device, such as a needle, such as a 22 gauge needle, a 25 gauge needle, a 27 gauge needle, and the like.

The total weight of microsphere in a single dosage an optimal amount, depending on the volume of the subconjunctival space and the activity or solubility of the protein. According to some embodiments, the dose is about 1 mg to about 40 mg of microspheres per dose. In some embodiments, the dose is between 0.1 mg to 1 mg, 10 mg to 30 mg, 10 mg to 20 mg, and the like. In an embodiment, a single injection may contain about 1 mg, or about 5 mg, or about 10 mg, or about 15 mg, or about 17 mg or about 20 mg, or about 30 mg, or about 35 mg of microspheres, including the incorporated therapeutic component. For non-human individuals, the dimensions and total weight of the microsphere(s) may be larger or smaller, depending on the type of individual.

An advantage that a poly(d,l-lactide-co-glycolide) ("PLGA") based microsphere formulation of protein has over conventional solution formulations is that the microsphere formulation can deliver the active protein over a longer period of time, in some cases over a matter of weeks or months.

### EXAMPLES

Without intending to limit the scope of the disclosure, example embodiments are set forth by the following Examples.

### EXAMPLE 1

Stabilization of a highly concentrated protein solution by the incorporation of excipients.

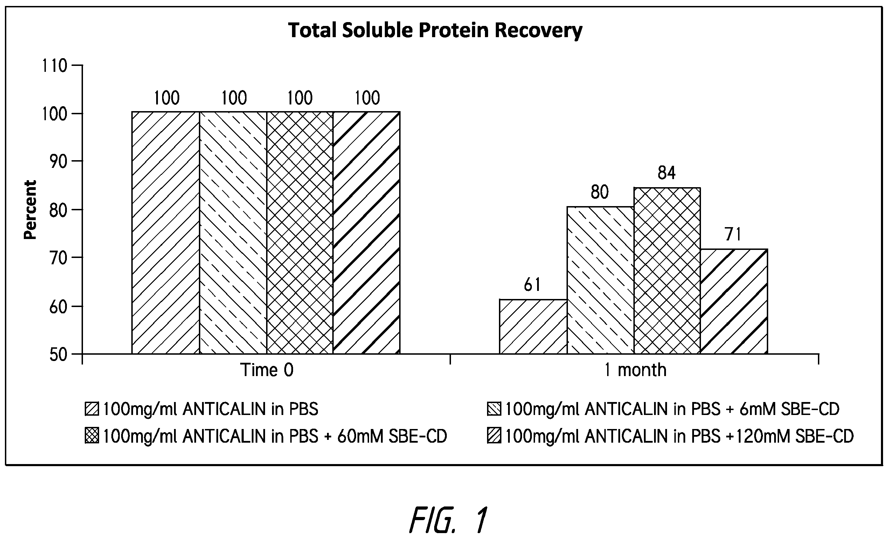

According to an example, the soluble protein recovery of 100 mg/ml anticalin example embodiment solutions formulated with and without excipients in phosphate buffered saline ("PBS") after one month at 37 °C was tested. According to first formulation, a 100 mg/mL solution of anticalin was prepared in PBS with SBE-CD having a molar concentration of 6 mM. The 6 mM SBE-CD was present in the formulation in an amount of 10.6 wt%, based on the total weight of the formulation. According to a second formulation, a 100 mg/mL solution of anticalin was prepared in PBS with 60 mM SBE-CD. The 60 mM SBE-CD was present in the formulation in an amount of 54.1 wt%, based on the total weight of the formulation. According to a third formulation, a 100 mg/mL solution of anticalin was prepared in PBS with 120mM SBD-CD. The 120 mM SBE-CD was present in the formulation an amount of 70.2 wt%, based on the total weight of the formulation. According to a first comparative formulation, a 100 mg/mL solution of anticalin was prepared in PBS with no SBE-CD. The solution was stored at 37 °C for one month, then analyzed to determine the percentage of soluble protein recoverable in the solution. The results are illustrated in Figure 1.

As shown in Figure 1, the first comparative formulation showed a 39% decline in soluble protein recovery after one month incubation at 37 °C. The decrease in soluble protein recover may have been attributed to protein aggregation leading to the formation of insoluble particulates. However, as shown in Figure 1, the addition of SBE-CD at a concentration of 6 mM and 60 mM to the solution of 100 mg/mL of anticalin in PBS improved the soluble protein recovery by 19% and 23%, respectively, after one month incubation at 37 °C. At the highest tested SBE-CD concentration of 120 mM, the improvement in soluble protein recovery as compared to the first comparative formulation with no SBE-CD is only 10%. Surprisingly, the highest percentage of soluble protein recovery occurs at the molar concentration of 60 mM SBE-CD, which was not the formulation tested with the highest molar concentration of SBE-CD. The first, second, third, and fourth formulations showed improved protein recovery compared to the formulation containing no additional excipients in PBS.

### EXAMPLE 2

Stabilization of Anticalin Solution at accelerated temperatures by the incorporation of excipients.

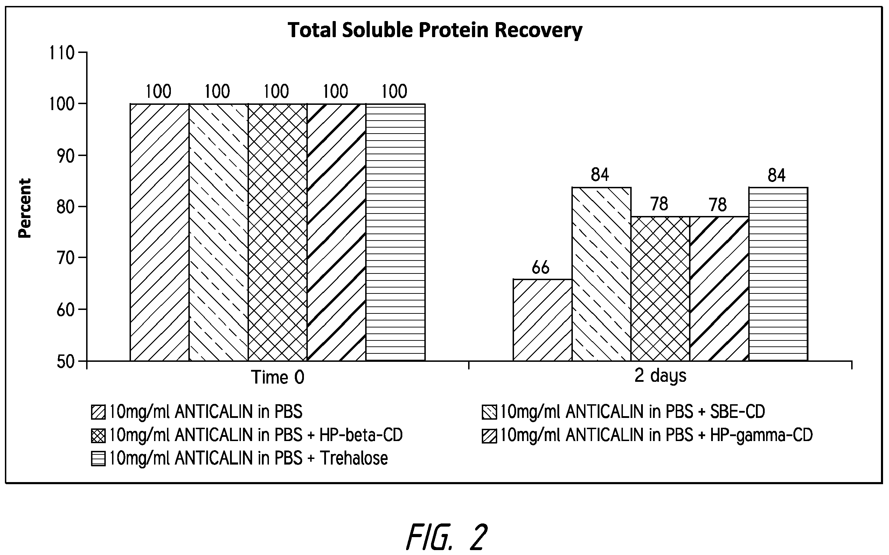

According to another example embodiment, the stability of an anticalin protein was evaluated at an accelerated temperature of 50 °C. According to this example, soluble protein recovery of 10 mg/ml anticalin example embodiment solutions formulated with and without excipients in phosphate buffered saline ("PBS") after two days at 50 °C was tested. According to a fourth formulation, a 10 mg/mL solution of anticalin was prepared in PBS with SBE-CD. The SBE-CD was present in the formulation in an amount of 46.0 wt%, based on the total weight of the formulation. According to a fifth formulation, a 10 mg/mL solution of anticalin was prepared in PBS with hydroxypropyl-beta-cyclodextrin ("HP-beta-CD"). The HP-beta-CD was present in the formulation in an amount of 35.2 wt%, based on the total weight of the formulation. According to a sixth formulation, a 10 mg/mL solution of anticalin was prepared in PBS with hydroxypropyl-gamma-cyclodextrin ("HP-gamma-CD"). The HP-gamma-CD was present in the formulation in an amount of 38.4 wt%, based on the total weight of the formulation. According to a seventh formulation, a 10 mg/mL solution of anticalin was prepared in PBS with trehalose. The trehalose was present in the formulation in an amount of 35.2 wt%, based on the total weight of the formulation. According to a second comparative formula, a 10 mg/mL solution of anticalin was prepared in PBS with no additional excipients. The solutions were stored in an oven maintained at 50 °C for two days, then analyzed to determine the percentage of soluble protein recoverable in the solution. The results are illustrated in Figure 2.

Figure 2 shows the soluble protein recovery of the 10 mg/mL anticalin solutions formulated with and without excipients in PBS after incubation in a 50 °C oven for two days. As illustrated in Figure 2, the soluble protein recovery of comparative formulation two was about 66%. As shown the addition of either SBE-CD (in the fourth formulation) or trehalose (in the seventh formulation) improved the soluble protein recovery by about 18%, while the addition of HP-beta-CD (in the fifth formulation) or HP-gamma-CD (in the sixth formulation) improved the soluble protein recovery by 12%. Surprisingly, the formulation including the carbohydrate trehalose exhibited as high soluble protein recovery as the formulation including SBE-CD. The fourth, fifth, sixth, and seven formulations all demonstrated improved protein recovery compared to the formulation that contained no additional excipients in PBS.

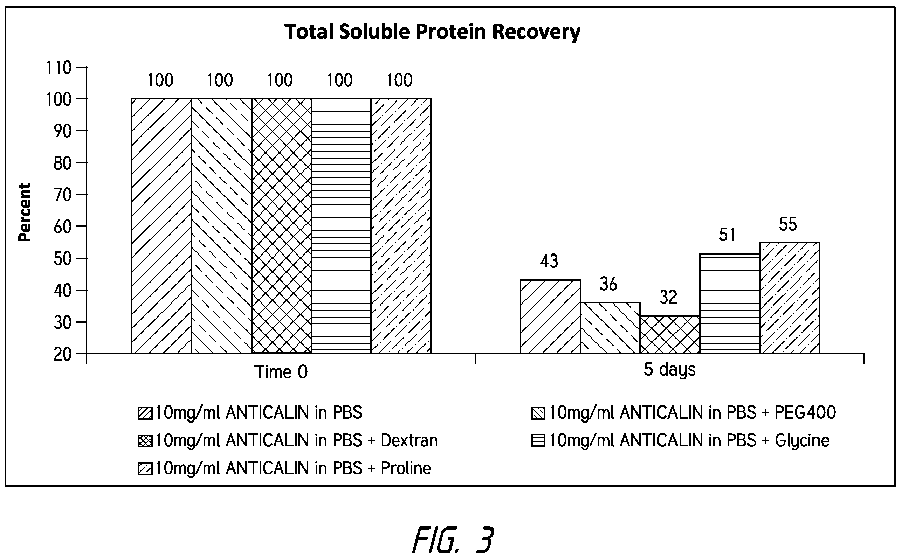

According to yet another example embodiment, the stability of an anticalin protein was evaluated at an accelerated temperature of 50 °C. According to this example, the soluble protein recovery of 10 mg/ml anticalin example embodiment solutions formulated with and without excipients in phosphate buffered saline ("PBS") after five days at 50 °C was tested. According to an eighth formulation, a 10 mg/mL solution of anticalin was prepared in PBS with PEG400. The PEG400 was present in the formulation in an amount of 54.6 wt%, based on the total weight of the formulation. According to a ninth formulation, a 10 mg/mL solution of anticalin was prepared in PBS with dextran. The dextran was present in the formulation in an amount of 63.7 wt%, based on the total weight of the formulation. According to a tenth formulation, a 10 mg/mL solution of anticalin was prepared in PBS with glycine. The glycine was present in the formulation in an amount of 4.8 wt%, based on the total weight of the formulation. According to an eleventh formulation, a 10 mg/mL solution of anticalin was prepared in PBS with proline. The proline was present in the formulation in an amount of 7.1 wt%, based on the total weight of the formulation. According to a third comparative formula, a 10 mg/mL solution of anticalin was prepared in PBS with no excipients. The solutions were stored in an oven maintained at 50 °C for five days, then analyzed to determine the percentage of soluble protein recoverable in the solution. The results are illustrated in Figure 3.

Figure 3 shows the soluble protein recovery of the 10 mg/mL anticalin solutions formulated with and without excipients in PBS after incubation in a 50 °C oven for five days. Here, the results show that not all excipients improved the soluble protein recovery, when compared with the protein solution without additional excipients. For example, the tenth and eleventh formulation, which included the addition of amino acids glycine and proline, respectively, increased the soluble recovery of anticalin. The addition of PEG 400 and dextran in the eighth and ninth formulations, respectively, decreased the soluble recovery of anticalin by 7%-11%.

### EXAMPLE 3

### Manufacturing and testing of PLGA-based Anticalin Microspheres

According to an example embodiment, PLGA-based anticalin microspheres were produced using a double emulsion solvent evaporation method. The method of making these microspheres is known as water-in-oil-in-water (W/O/W) process, and is disclosed, for example, in PCT Publication No. 2011/041642, the methods of making microspheres disclosed therein are hereby expressly incorporated by reference. According to this example, the process was executed at a temperature around 4 °C to 10 °C to improve batch to batch reproducibility and increase the stability of the anticalin.

In order to determine the experimental loading of the protein within the PLGA polymer network and the quality of the protein, the encapsulated protein, and its soluble degradants were extracted from the dried microspheres using a two-step extraction method followed by SEC analysis. Degradation products of anticalin were classified into two categories: high molecular weight ("HMW") species and low molecular weight ("LMW") species. HMW species are aggregates of anticalin with molecular weight higher than that of the monomer of anticalin, whereas LMW species are fragments of anticalin with molecular weight lower than that of the anticalin monomer.

The release profiles of the prepared microspheres were evaluated in vitro using a release medium comprised of 100mM phosphate, pH 7.4 at a temperature of 37 C with no agitation. All samples were prepared in duplicates. At each pre-set timepoint, 90% of the solution was sampled for analytical analysis, and the sampled volume was replaced with the same volume of fresh release medium.

### EXAMPLE 4

### Encapsulation of Anticalin in PLGA-based microspheres

Table B, below, illustrates several example formulations of anticalin in PLGA-based microspheres. As shown in Table B, PLGA-based microsphere formulations without any excipients have an encapsulation efficiency of around 78.6%, which includes 11.4% of the soluble aggregates. As is shown in the table, advantageously, addition of select excipients reduced the soluble aggregate composition from 11.4% to as low as 3.9%.

**TABLE B**

| The Effect of Excipients in PLGA Microsphere Formulations Containing 6% Anticalin | | | | | | | |
|---|---|---|---|---|---|---|---|
| Example No. | Resomer® RG755S (%wt) | Protein, Target (%wt) | Stabilizer(s) | | | Encapsulation Efficiency ("EE"), Total Protein (%) | Aggregates (%wt) |
| | | | SBE-CD Target Load (%wt) | Arginine Target Load (%wt) | Trehalose Target Load (%wt) | | |
| CE 1 | 93 | 7 | 0.0 | 0.0 | 0.0 | 78.6 | 11.4 |
| EX 1 | 85 | 6 | 6.5 | 1.6 | 0.0 | 80.5 | 3.9 |
| EX 2 | 85 | 6 | 0.0 | 1.6 | 6.5 | 74.3 | 7.3 |
| EX 3 | 87 | 6 | 6.5 | 0.0 | 0.0 | 65.4 | 4.7 |
| EX 4 | 87 | 6 | 0.0 | 0.0 | 6.5 | 82.6 | 6.2 |
| EX 5 | 91 | 7 | 0.0 | 1.6 | 0.0 | 84.8 | 10.7 |

As illustrated in Table B, the formulations that included SBE-CD had the lowest amount of soluble aggregates at a range of 3.9% to 4.7% [a three-fold reduction of aggregates]. While not wishing to be bound by any theory, this result may demonstrate the ability of SBE-CD to protect the protein from the microsphere process conditions and release conditions. Interestingly, while the addition of arginine alone had little effect of reducing soluble aggregates, it improved encapsulation efficiency of the protein when used alone or in combination with SBE-CD. Trehalose also demonstrated a reduction of aggregates in microsphere formulations. Surprisingly, the combination of SBE-CD and arginine was synergistic in reducing aggregates and maximizing encapsulation efficiency in the process of preparing PLGA microspheres.

Table C illustrates a broader application of this combination, in minimizing aggregates, where incorporating these excipients in other PLGA polymer combinations produce microspheres with aggregate concentrations around 5%.

**TABLE C**

| Low Aggregation Formulation of SBE-CD/Arginine-loaded Microsphere-Containing 6% Anticalin | | | | | | | | |
|---|---|---|---|---|---|---|---|---|
| Example No. | Resomer ® RG755S | Resomer ® RG502H | Resomer ® R202H | Resomer ® RGP d5055 | Protei n (%wt) | Excipient s | EE, Total (%wt ) | Aggregate s (%wt) |
| CE 2 | 81% | 0% | 0% | 12% | 6 | None | 90.8 | 10.4 |
| EX 6 | 76% | 0% | 0% | 11% | 6 | 6.5% SBE-CD + 1.6% Arginine | 81.7 | 5.8 |
| EX 7 | 80% | 6% | 0% | 0% | 6 | 6.5% SBE-CD + 1.6% Arginine | 70.1 | 5.4 |
| EX 8 | 80% | 0% | 6% | 0% | 6 | 6.5% SBE-CD + 1.6% Arginine | 72.2 | 4.9 |

### EXAMPLE 5

### Sustained release of Anticalin from PLGA-based microspheres

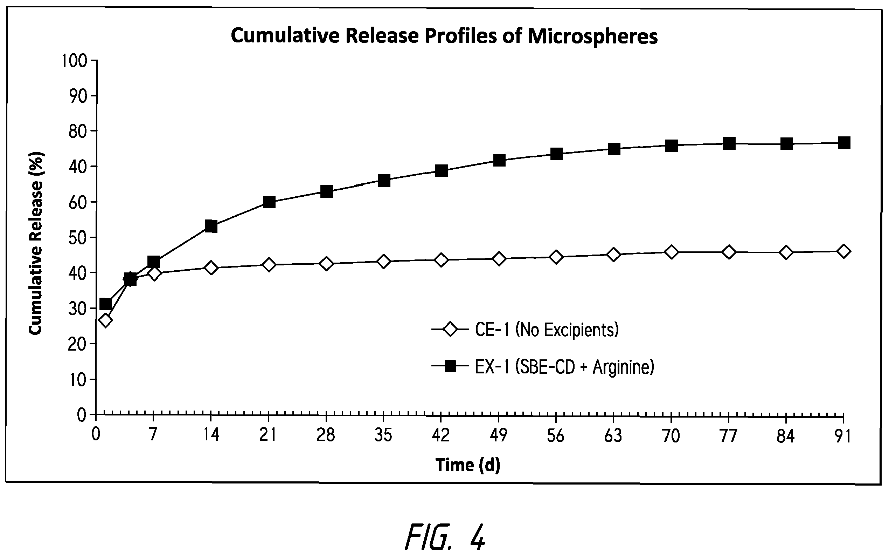

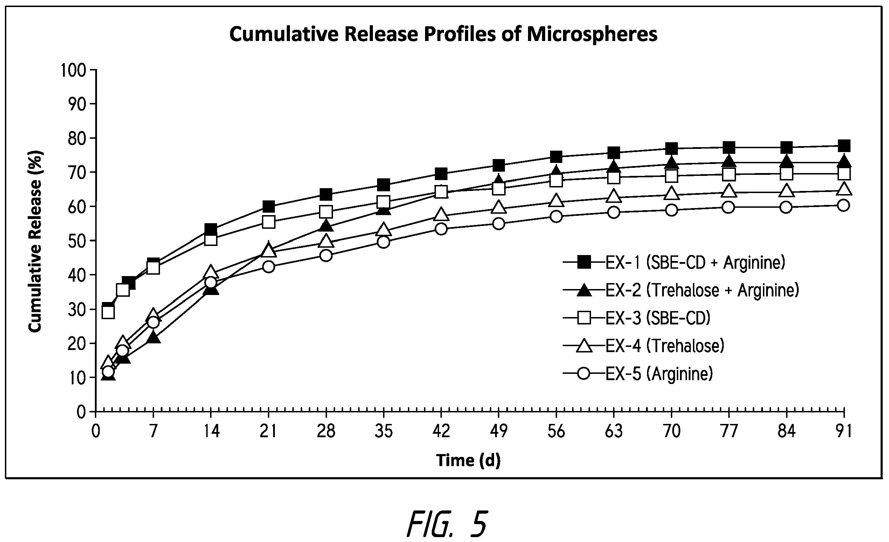

PLGA-based microspheres were fabricated and tested as described in Example 3. Figures 4 and 5 display different release profiles for the anticalin and demonstrate the ability to modulate the release of the anticalin to target different burst, release rates, and durations by the modification of the microsphere composition and fabrication process.

Figures 4 and 5 demonstrate some of the beneficial properties resulting from the incorporation of certain excipients into the PLGA-based microspheres, for example, the ability of formulations to achieve sustained release for two to three months. The microsphere formulations with the excipients showed a steady continuous release, whereas the release rate of the microsphere formulation without the excipients quickly slowed down after seven days, as illustrated in Figure 4. The PLGA-based microspheres with excipients exhibited almost complete recovery with a cumulative release of about 77%, whereas the PLGA-based microspheres without excipients showed a cumulative release of about 47%. Figure 5 illustrates the ability to modulate burst and release rate using different excipients with the same process parameters.

### EXAMPLE 6

### Effect of polymers on release of anticalin from PLGA-based microspheres

Polymer properties such as inherent viscosity ("IV"), glass transition temperature, and molecular weight can facilitate the degree of protein encapsulation and release. Three polymers with different IV (Resomer ® RG 755S, RG 753S, and RG 752S) were evaluated in microsphere formulation. Table D illustrates the effect of polymer inherent viscosity on encapsulation and burst. The table shows that although the same process and the same excipient composition were used, the microsphere formulation with RG755S was shown to have the highest encapsulation efficiency, the highest protein quality (e.g. the lowest percentage of soluble aggregates), and the lowest burst.

**TABLE D**

| Effect of Polymer Inherent Viscosity on Encapsulation and Burst | | | | | | |
|---|---|---|---|---|---|---|
| Example No. | Formulation | Polymer Inherent Viscosity (dL/g) | Polymer End Group | EE Total (%wt) | Soluble Aggregates (%wt) | 1-Day Burst (%wt) |
| EX 9 | RG 755S/ Anticalin/Buffer/SBE-CD/ Arginine | 0.50-0.70 | -OCH3 | 65.8 | 3.8 | 38.8 |
| EX 10 | RG 753S/ Anticalin/Buffer/SBE-CD/ Arginine | 0.32-0.44 | -OCH3 | 62.3 | 4.9 | 52.3 |
| EX 11 | RG 752S/ Anticalin/Buffer/SBE-CD/ Arginine | 0.16-0.24 | -OCH3 | 22.2 | 8.4 | 103.9 |

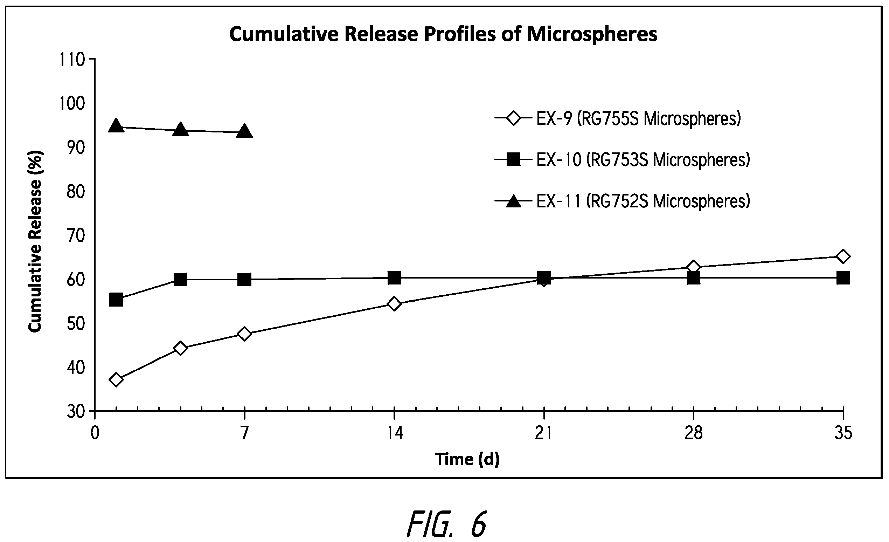

Figure 6 illustrates the release of anticalin from microspheres fabricated from polymers with different IV. The microspheres fabricated from RG755S, which has the highest IV, showed continuous release whereas the microspheres generated from RG753S and RG752S showed flattened release curves after seven days.

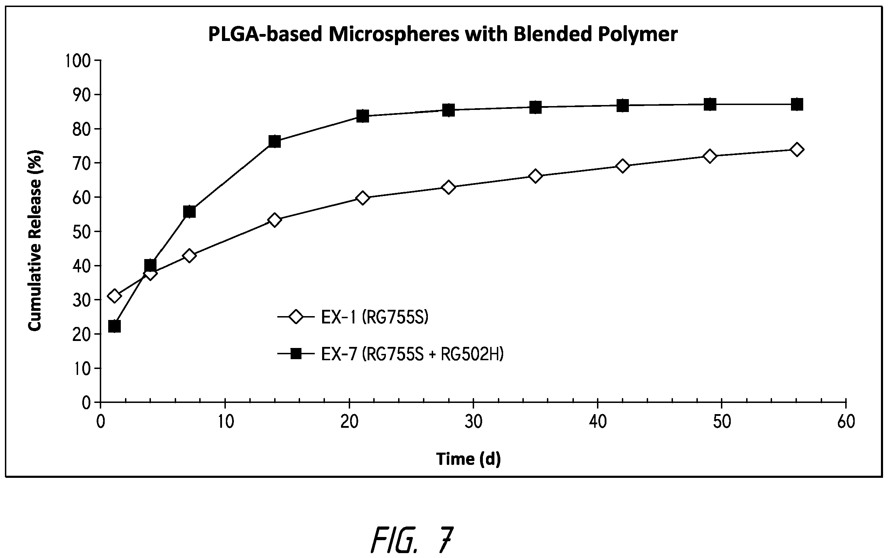

Polymer blending can affect the burst and the release rate of anticalin from PLGA-based microspheres. As illustrated in Figure 7, RG755S can be blended with a more hydrophilic polymer to lower the burst and increase the release rate of anticalin from the microspheres. The polymer blend of RG755S and RG502H at a ratio of 13 to 1 resulted in a microsphere formulation with a lower burst and faster release rate than the microsphere formulation with mainly RG755S. However, the release duration was reduced to one month.

### EXAMPLE 7

### Binding activity of released anticalin from PLGA-based microspheres

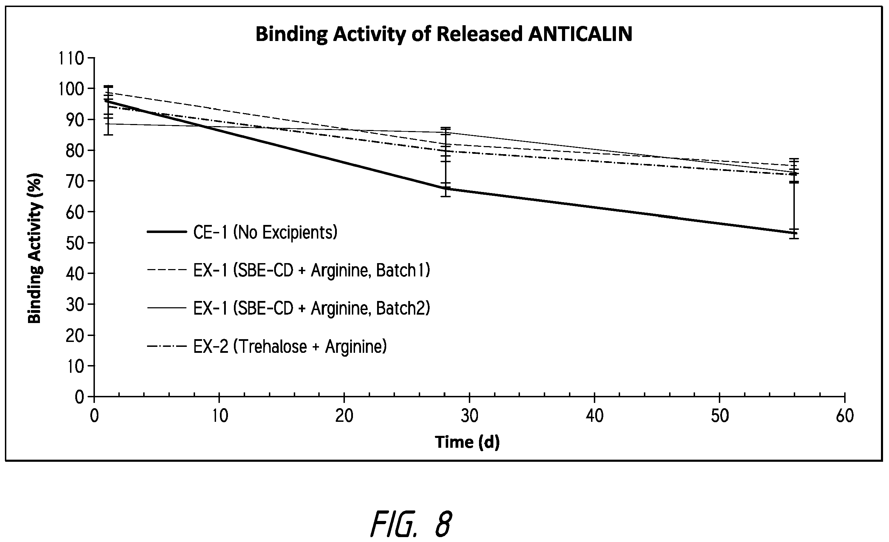

The binding activity of the anticalin released from select PLGA-based microspheres was measured by an enzyme-linked immunosorbent assay ("ELISA"). The activity is defined as the percentage of concentration determined by the ratio of the ELISA concentration to the SEC concentration. Figure 8 illustrates the binding activity of the released anticalin from PLGA-based microspheres for the duration of two months. The release protein is active throughout the release period with binding activity higher than 70% for the microsphere formulations with excipients and higher than 50% for the microsphere formulations without excipients.

### EXAMPLE 8

### Manufacturing, testing, and release of PLGA-based microspheres containing antibody

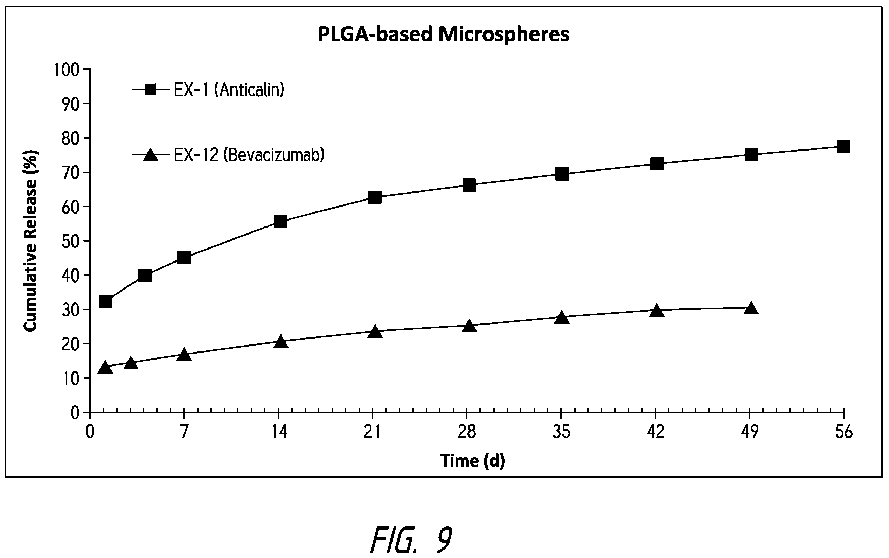

According to yet another example, PLGA-based microspheres were fabricated and tested as described in Example 3 using an antibody, bevacizumab, instead of the anti-VEGF protein, anticalin. Figure 9 compares the release profiles of a PLGA-based microsphere formulation with anticalin and a PLGA-based microsphere formulation with bevacizumab. The data demonstrates that the process and formulation that were utilized to develop a sustained release formulation for delivering anticalin can be applied to a full-length monoclonal antibody with molecular weight of 149 kDa to delivery active proteins for a sustained period of time.

Although this invention has been disclosed in the context of certain preferred embodiments and examples, it will be understood by those skilled in the art that the present invention extends beyond the specifically disclosed embodiments to other alternative embodiments and/or uses of the invention and obvious modifications and equivalents thereof. In addition while the number of variations of the invention have been shown and described in detail, other modifications, which are within the scope of this invention, will be readily apparent to those of skill in the art based on this disclosure. It is also contemplated that various combinations or subcombinations of the specific features and aspects of the embodiments can be made and still fall within the scope of the invention. Accordingly, it should be understood that various features and aspects of the disclosed embodiments can be combined with, or substituted for, one another in order to perform varying modes of the disclosed invention. Thus, it is intended that the scope of the present invention herein disclosed should not be limited by the particular disclosed embodiments described above, but should be determined only by a fair reading of the claims.

## Claims

1. A composition of matter comprising:
particles, the particles comprising a protein, a biodegradable polymer, and one or more excipients;
wherein the particles are configured to deliver the protein for a period of time of not less than two months.

2. The composition of Claim 1, wherein the biodegradable polymer comprises poly(d,l-lactide-co-glycolide), poly(d,l-lactide), and/or polyethylene glycol copolymers with an inherent viscosity in the range of 0.1 dL/g to 1.0 dL/g.

3. The composition of Claim 1, wherein the protein has a molecular weight in the range of 10 kDa to 150 kDa.

4. The composition of Claim 1, wherein the one or more excipients comprise cyclodextrin, trehalose, arginine, or a combination thereof.

5. The composition of Claim 4, wherein the one or more excipients comprise cyclodextrin, and the cyclodextrin is sulfobutylether-beta-cyclodextrin.

6. An aqueous formulation comprising:
a protein;
a buffer; and
one or more excipients selected from the group consisting of cyclodextrin, trehalose, and arginine;
wherein the one or more excipients are present in the aqueous formulation at a molar ratio in the range of 10/1 to 200/1, with respect to the protein molar concentration.
